# EUROPEAN PATENT SPECIFICATION

(11) **EP 4 452 114 B1**
(45) Date of publication and mention of the grant of the patent: **27.05.2026**
(21) Application number: 22843159.9
(22) Date of filing: 20.12.2022
(51) Int. Cl.: A61B 34/10, A61B 34/20

(54) **PCL BALANCING IN KNEE REPLACEMENT**
PCL-BALANCING BEI KNIEERSATZ
ÉQUILIBRAGE DE PCL LORS DU REMPLACEMENT DU GENOU

(30) Priority: 20.12.2021 GB 202118561
(43) Date of publication of application: 30.10.2024
(73) Proprietor: DePuy Ireland Unlimited Company, Ringaskiddy, County Cork (IE)
(72) Inventor: ROCK, Michael, Leeds Yorkshire LS11 8DT (GB)
(74) Representative: Murgitroyd & Company
(86) International application number: PCT/EP2022/086920
(87) International publication number: WO 2023/118090

(56) References cited:
- US-A1- 2005 119 661
- US-A1- 2006 241 405
- US-A1- 2007 198 022

## Description

### TECHNICAL FIELD

The present disclosure relates to data processing apparatus, data processing methods and methods for use in knee replacement surgical procedures.

Knee replacement surgery or knee arthroplasty is a generally well known surgical procedure in which one compartment (unicompartmental knee arthroplasty or partial knee replacement) or the whole of the knee (total knee replacement) is replaced by prosthetic implants typically including a femoral component and a tibial component. The femoral component is attached to the distal end of the femur and generally replaces the femoral condyles. The tibial component is attached to the proximal end of the tibia and generally replaces the tibial plateau.

An important feature of knee arthroplasty generally is the size, position and orientation of the prosthetic components. This generally involves determining the positions and orientations of the various cuts made to resect the distal femur and the proximal tibia and is generally known in the art.

A further important feature of knee arthroplasty is management of the soft tissues that surround the knee and which can be an important aspect of the surgical outcome for the patient. During some knee procedures, some of the native soft tissue structures may be sacrificed as part of the surgical procedure, such as the anterior cruciate ligament. In some instances, surgeons may perform soft tissue release to reduce the tension in the soft tissues so as to improve the overall performance of the knee joint. In other instances, surgeons may adjust cut positions and/or use shims or spacers to take up some of the laxity that would otherwise be present in the soft tissue structures.

However, the knee joint is not purely a simple pivot about a constant axis of rotation and involves quite a complex relative movement between the articulating surfaces of the femur and tibia during which there is a complex interaction of the various forces exerted on the knee by the various soft tissue structures.

Hence, apparatus and methods which help take into account the soft tissue structures of the knee during replacement knee surgery would be beneficial.

US 2005/119661 A1 discloses a method to quantitatively determine the degree of soft tissue constraints in knee arthroplasty based on either a two ligament model (medial collateral and lateral collateral ligaments) or a three ligament model (including the posterior cruciate ligament). Tibial displacement is measured under various positions and is used to estimate ligament attachment sites and neutral ligament lengths.

### SUMMARY

The present invention is solely defined in the appended claims.

According to a first aspect of the invention, there is provided a non-transitory computer readable medium storing instructions executable by a data processor to carry out a data processing method comprising the following steps:
(i) determining a femoral attachment position of a posterior cruciate ligament to a femur of a knee of a patient;
(ii) determining a tibial attachment position of the posterior cruciate ligament to a tibia of the knee of the patient;
(iii) determining a displacement of the tibia relative to the femur along an anterior-posterior axis of the tibia of the patient for each of a plurality of knee angles, corresponding to a posterior drawer test being applied to the knee of the patient;
(iv) determining the position of the femur and the tibia of the knee of the patient arising from a planned femoral implant position and a planned tibial implant position for each of the plurality of knee angles;
(v) determining a maximum length of the posterior cruciate ligament as the distance between the femoral attachment position and the tibial attachment position for each of the plurality of knee angles using the determined displacement, the femoral attachment position and the tibial attachment position;
(vi) determining a rollback of the femur relative to the tibia along an anterior-posterior axis of the tibia for each of the plurality of knee angles;
(vii) determining a required length of the posterior cruciate ligament for each of the plurality of knee angles based on (a) the distance between the femoral attachment position and the tibial attachment position for each of the plurality of knee angles using the determined positions of the femur and the tibia arising from the planned femoral implant position and the planned tibial implant position, the femoral attachment position, and the tibial attachment position, and (b) an amount of rollback required to prevent the required length of the posterior cruciate ligament exceeding the maximum length of the posterior cruciate ligament; and
(viii) outputting a comparison of the required length of the posterior cruciate ligament and the maximum length of the posterior cruciate ligament as a function of knee angle.

The rollback may correspond to a rollback of the knee when a range of motion test was applied to the native knee of the patient.

The rollback may correspond to a fixed amount of roll back of the knee.

The data processing method may further comprise:
i.receiving a change to the planned femoral implant position and/or a change to the planned tibial implant position, and repeating (v), (vi), (vii) and (viii) using the changed planned femoral implant position and/or the changed planned tibial implant position.

The amount of rollback may be determined for a femoral flexion angle of at least 50° or at least 60°.

Outputting the comparison of the required length of the posterior cruciate ligament and the maximum length of the posterior cruciate ligament as a function of knee angle may comprise outputting a graphical display of the required length of the posterior cruciate ligament and the maximum length of the posterior cruciate ligament as a function of knee angle.

The plurality of knee angles nay be within the range of 0° of flexion to 120° of flexion.

The plurality of knee angles nay be within the range of 60° of flexion to 120° of flexion.

The plurality of knee angles may be within the range of 60° of flexion to 90° of flexion.

The number of the plurality of knee angles may be at least three.

A second aspect of the present invention relates to a data processing apparatus comprising: a processor; and the non-transitory computer readable medium of the first aspect.

A third aspect of the present invention relates to a computer assisted surgery system including the data processing apparatus of the second aspect.

The computer assisted surgery system may further comprise: a tracking system; and/or a surgical robot.

A further illustrative aspect of the present disclosure which does not form part of the present invention relates to a method comprising:
(i) measuring a loaded displacement between a tibia of a knee of a patient and a femur of the knee in an anterior-posterior direction of the tibia at a plurality of knee angles of the knee for a posterior force applied to the tibia;
(ii) measuring an unloaded, along the anterior-posterior direction, displacement between the tibia and the femur in the anterior-posterior direction of the tibia at a plurality of knee angles of the knee without a force applied to the tibia;
(iii) determining an estimate of the maximum length of the posterior cruciate ligament for the plurality of knee angles using the loaded displacement;
(iv) determining a planned knee joint separation between the tibia and the femur for a planned tibial implant position and a planned femoral implant position for the knee of the patient and said unloaded displacement between the tibia and the femur at a plurality of knee angles of the knee of the patient;
(v) determining an estimate of the required length of the posterior cruciate ligament for the plurality of knee angles from the determined planned knee joint separation;
(vi) outputting a comparison of the estimate of the required length of the posterior cruciate ligament and the estimate of the maximum length of the posterior cruciate ligament as a function of knee angle for the knee of the patient.

The method may further comprise preparing the knee of the patient for a knee laxity test before measuring the loaded displacement.

Preparing the knee of the patient may include one or more of: removing osteophytes; removing meniscus; removing the anterior cruciate ligament; posterior capsular release.

The method may further comprise:
(i) tracking the position of the femur and the position of the tibia of the patient;
   determining the knee angle from the tracked position of the femur and the tracked position of the tibia; and/or
(ii) measuring the loaded displacement from the tracked position of the femur and the tracked position of the tibia; and/or
(iii) measuring the unloaded displacement from the tracked position of the femur and the tracked position of the tibia.

The method may further comprise:
(i) determining a femoral attachment position of the posterior cruciate ligament to the femur; and/or
(ii) determining a tibial attachment position of the posterior cruciate ligament to the tibia.

The femoral attachment position and/or the tibial attachment position may be used to determine the estimate of the required length of the posterior cruciate ligament.

A trackable instrument may be used to determine the femoral attachment position of the posterior cruciate ligament to the femur.

The tibial attachment position of the posterior cruciate ligament to the tibia may be determined from an estimate of the attachment position of the posterior cruciate ligament to the tibia.

Outputting the comparison of the estimate of the required length of the posterior cruciate ligament and the estimate of the maximum length of the posterior cruciate ligament may comprise displaying a graph of the estimate of the required length of the posterior cruciate ligament and the estimate of the maximum length of the posterior cruciate ligament as a function of knee angle.

The method may further comprise:
(i) modifying the planned tibial implant position and/or modifying the planned femoral implant position;
(ii) re-determining the planned knee joint separation between the tibia and the femur for the modified planned tibial implant position and/or the modified planned femoral implant position for the knee of the patient and said unloaded displacement between the tibia and the femur at a plurality of knee angles of the knee of the patient;
(iii) re-determining the estimate of the required length of the posterior cruciate ligament for the plurality of knee angles from the re-determined planned knee joint separation;
(iv) outputting a comparison of the re-determined estimate of the required length of the posterior cruciate ligament and the estimate of the maximum length of the posterior cruciate ligament as a function of knee angle for the knee of the patient.

The plurality of knee angles may include at least three different knee angles.

The plurality of knee angles may range from 0° to 120° of extension.

The plurality of knee angles may range from 60° to 120° of extension.

The plurality of knee angles may range from 60° to 90° of extension.

There may be an interval of at least 10, 15, 20 or 30 degrees between successive knee angles of the plurality of knee angles.

The method may further comprise:
(i) determining an amount of rollback required to prevent the estimate of the required length of the posterior cruciate ligament exceeding the estimate of the maximum length of the posterior cruciate ligament; and
(ii) outputting the amount of rollback.

The amount of rollback may be determined for one or more femoral flexion angles of at least 60°.

The unloaded displacement may be measured during a passive range of motion test of the native knee of the patient.

The method may be carried out using a computer assisted surgery system and wherein the computer assisted surgery system is used to carry out a knee replacement procedure on the patient using the planned tibial implant position and/or the planned femoral implant position.

The computer assisted surgery system may include a surgical robot and wherein the surgical robot is used to carry out at least some of the knee replacement procedure on the patient using the planned tibial implant position and/or said planned femoral implant position.

The planned tibial implant position may include one or more of: tibial resection position; tibial slope; and tibial implant thickness; and/or wherein the planned femoral implant position may include one or more of: distal femoral position; and anterior-posterior femoral position

### BRIEF DESCRIPTION OF THE DRAWINGS

Embodiments of the disclosure will now be described in greater detail, and by way of example only, and with reference to the accompanying drawings, in which:
(i) Figure 1 shows a schematic block diagram of a system including data processing apparatus according to an embodiment of a first aspect of the disclosure;
(ii) Figure 2 shows a flow chart illustrating a method according to an embodiment of a second aspect of the disclosure;
(iii) Figure 3 shows a process flow chart illustrating a data processing method according to an embodiment of a third aspect of the disclosure;
(iv) Figure 4 shows a schematic illustration of the sagittal plane of a knee illustrating movement of the tibia relative to the femur generally along the anterior-posterior axis;
(v) Figure 5 shows a schematic illustration of the sagittal plane of a knee illustrating extension of the Posterior Cruciate Ligament (PCL) owing to displacement of the tibia relative to the femur generally along the anterior-posterior axis;
(vi) Figure 6 shows a first graphical output of the data processing method of Figure 3 and illustrating the maximum available PCL length and the required PCL length for a planned knee implant position and different amounts of rollback as a function of knee angle;
(vii) Figure 7 shows a second graphical output of the data processing method of Figure 3 similar to Figure 6 but over a smaller range of knee angle; and
(viii) Figure 8 shows a third graphical output of the data processing method of Figure 3 illustrating the difference in PCL length between the maximum available PCL length and the required PCL length for each of the different amounts of rollback graph over the same range of knee angle as Figure 6.

### DETAILED DESCRIPTION OF THE DRAWINGS

In the Figures of drawings, the same reference numerals are used to refer to like items unless indicated otherwise.

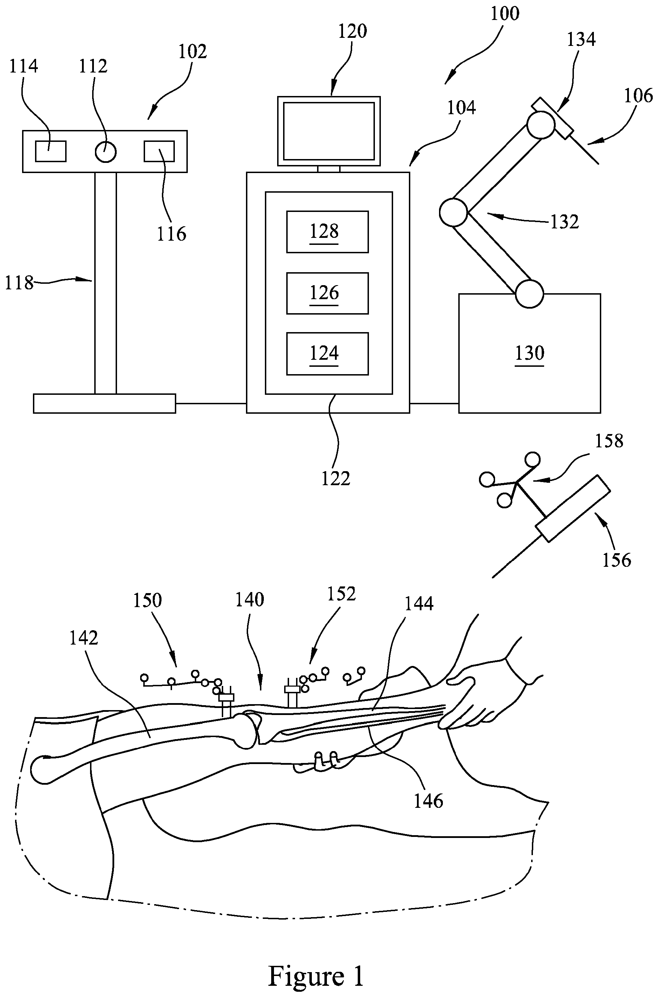

With reference to Figure 1 there is shown a schematic block diagram of a computer assisted surgery (CAS) system 100 according to an aspect of the disclosure and which may include apparatus and perform methods according to other aspects of the disclosure. The CAS system 100 may include a tracking system 102, a data processing system 104 and, optionally, a surgical robot 106. Although illustrated as separate subsystems in Figure 1, it will be appreciated that tracking system, data processing system and robotics system 106 may be integrated into a single CAS system.

In the illustrated embodiment, the tracking system 102 is provided in the form of an infrared optical tracking system including a source of infrared radiation 112, a pair of mutually spaced infrared cameras, 114, 116, mounted on a support 118. However, other wired and wireless tracking systems are also generally known in the art and may utilise, for example, acoustic, magnetic and other radiation based tracking technologies. However, simply for the sake of clarity of explanation, an infrared wireless tracking system will be described. The tracking system 102 is in communication with the data processing system 104.

The data processing system 104 includes substantially conventional data processing apparatus, including one or more data processors, memory and storage devices together with various communication buses, power supplies, communication interfaces and user input / output interfaces and devices. The data processing apparatus is configured by suitable software stored in memory to carry out various operations as described in greater detail below. As schematically illustrated in Figure 1, the data processing system 104 includes a display 120. Display 120 may display various images in the form of data and graphics to the user during use. In some embodiments, display 120 may be a touch screen device to provide user input to control operation of the CAS system 100.

Data processing system 104 includes software 122 which may include one or more modules as schematically illustrated in Figure 1. The data processing system 104 may include tracking software which receives data and/or tracking signals from the tracking system and converts those into position and / or orientation data specifying the position and / or orientation of various items tracked by the tracking system within a common reference frame of the tracking system. The position and / or orientation data may be passed by the tracking software subsystem to other parts of the CAS system as required. The CAS software 122 may include a workflow module 124 which controls the overall operation and workflow of the CAS system 100. The workflow module 124 may be used to select a surgical procedure to be carried out, a particular version of that surgical procedure and also various modifications to, or specific instances of, a surgical procedure.

A planning module 126 may also be provided by which the user may plan various aspects of the surgical procedure. For example, for an orthopaedic surgical procedure, this may include planning the type of implant to be used, a size of the implant, a position and / or orientation of the implant and the position and / or orientation of various cuts to be made to the bones of the patient in order to implement the surgical plan. In other instances, planning may be carried out using a separate application and the planning data imported to the CAS system over a network connection or similar. For total knee replacement, the planning data may include one, some or all of: the position or depth of the distal femoral cut; the femoral anterior-posterior position; the distal femoral valgus angle; the femoral internal/external rotation; the proximal tibial resection level or height; and the tibial varus angle.

The software 122 may also include a registration module 128 which receives tracking data from the tracking system in order to register the position of the patient's bones within the reference frame of the tracking system. Example registration methods for knees are described in the Attune Knee System, Knee3 Surgical Technique of DePuy Synthes. Registration is generally known in the art and so is not described in the greater detail herein.

As also illustrated in Figure 1, the CAS system 100 may optionally include a surgical robot 106. Surgical robot 106 includes a base 130 including various electronic controls, interface and power systems. An articulating limb or arm 132 is attached to the base 130 and includes an end effector or instrument 134. Multiple articulating limbs may be provided. The surgical robot 106 may be controlled by the data processing system 104 in order to carry out one or more steps of the surgical procedure under control of the surgical workflow software 124 and a user.

As also illustrated in Figure 1, there is shown a view of a right knee joint 140 of a patient, generally parallel to the sagittal plane, including the femur 142, tibia 144 and fibular 146. The patella is omitted for the sake of clarity. A first reference array 150 including an arrangement of three infrared reflective spheres is shown attached to the femur. A second reference array 152 similar to the first, is shown attached to the tibia. Also illustrated in Figure 1 is a trackable pointer instrument 156 bearing a third reference array 158. Trackable pointer 156 can be used to capture the position of various anatomical features of the patient's knee during the surgical procedure as generally known in the art.

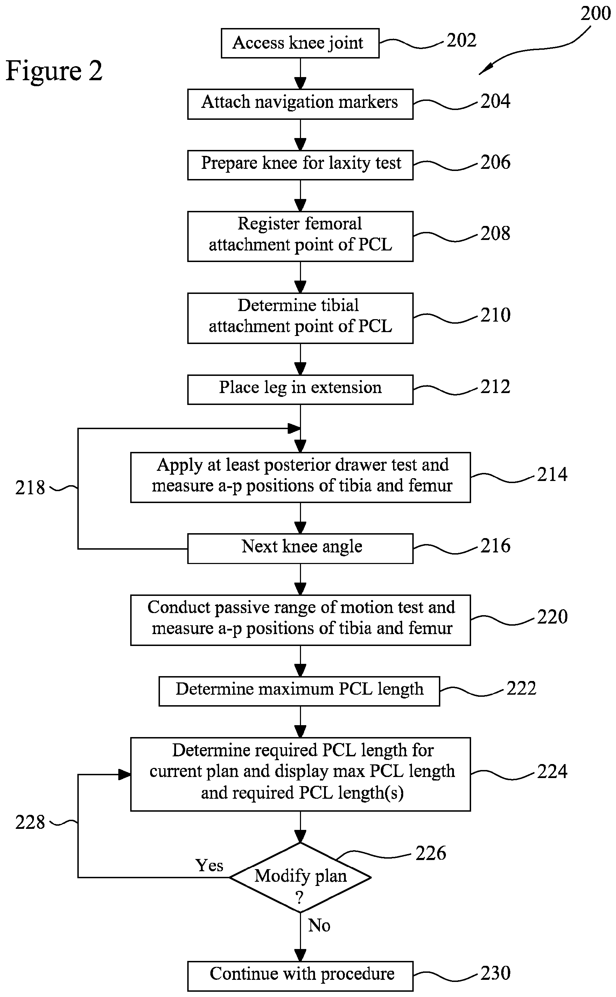

Figure 2 shows a flow chart illustrating a method 200 according to an aspect of the disclosure. Method 200 is used to generate anterior-posterior soft tissue balancing data for the patient's knee as a function of knee angle. In particular, method 200 is used to measure the Posterior Cruciate Ligament (PCL) length as a function of knee angle and then to use that to assist a surgeon in assessing and/or modifying a plan for the knee implants. At 202, the surgeon creates access to the knee joint as is generally known in the art. At 204, a first navigation marker 150 is attached to the femur and a second navigation marker 152 is attached to the tibia 144. These allow the position and orientation of the femur 142 and the tibia 144 to be tracked during the surgical procedure. The order of accessing the knee joint and attaching the navigation markers may be reversed and is not important.

Previously, a surgical plan has been created for the knee joint. This may include a planned height and varus angle for a proximal tibial cut. Also, the planned position of the distal femoral resection, femoral anterior-posterior position, femoral valgus angle and femoral internal/external rotation may have been planned. These may be planned using planning software based on images of the patient's femur and tibia or models thereof.

At 206, the exposed knee joint is prepared for a knee laxity test. Preparation may include, for example, one or more of removing any osteophytes, removing the meniscus where possible, removing the Anterior Cruciate Ligament (ACL) if a non ACL preserving knee implant system is being used, and releasing the posterior medial capsule, if required.

At some stage the surgeon registers the femur and the tibia within the reference frame or co-ordinate system of the tracking system. This may include using the trackable pointer 156 to capture the positions of various anatomical features on the femur and the tibia so that the femoral anatomical axis and the tibial anatomical axis may be defined and also the major anatomical axes and hence anatomical planes of the patient, i.e. the anterior-posterior axis, the medial-lateral axis and the inferior-superior axis. Registration of the femur and tibia may be done after 206, but more usefully may be done between 204 and 206 as this allows the surgeon to have navigation data available when preparing the knee for the laxity test at 206. This may improve the ability to detect osteophytes and any ligament tightness that may require releases.

Then at 208, the position at which the PCL attaches to the femur is determined. This may be achieved by tracking the position of the trackable pointer 156 in order to register one or more positions within the reference frame of the tracking system. For example, the trackable pointer 156 may be used to register a central point of the PCL at which it attaches to the femur. Alternatively or additionally, the trackable pointer 156 could be used to draw around the PCL where it attaches to the femur to capture the shape of that part of the surface of the femur and then a software routine may be used to determine the centroid of the shape. The centroid of the shape may then be set as the attachment point of the PCL to the femur.

After the attachment point of the PCL to the femur has been captured at 208, the attachment point of the PCL to the tibia is determined. As will be appreciated the PCL attaches toward the posterior of the tibia. A number of different methods may be used to determine the position of the attachment point of the PCL to the tibia at 210.

In some case, depending on access to the knee, it may be possible to register the attachment point directly using the trackable pointer 156. However, in other circumstances, it may not be possible to use the trackable pointer.

An estimation of the attachment point may be made using a software routine. Medial and lateral points on the proximal tibia may be captured using the trackable pointer and then the medial-lateral centre of the tibia is calculated. For a tibial implant size corresponding to the medial-lateral size of the tibia, a most posterior point of the tibial implant at the centre of the medial-lateral axis may be identified. The PCL attachment point is below the joint line by approximately 1cm. Therefore, the PCL attachment point may be set approximately 10mm distally from the identified point and used as the tibial PCL attachment point.

Alternatively, the most posterior point on the anterior-posterior axis of the tibia can be registered using the trackable pointer 156 and identified. Again, the PCL attachment point is below the joint line by approximately 1cm. Therefore, the PCL attachment point may be set approximately 10mm distally from the identified point and used as the tibial PCL attachment point.

An imaging method could be used in which a pre-operative image of the patient's knee which shows the attachment point of the PCL to the tibia is registered with reference frame of the tracking system, for example by using the trackable pointer 156 to capture various anatomical features of the tibia also shown in the image. For example, if the tibial PCL attachment point can be identified from an MRI scan and the MRI scan is registered with the patient's tibia intra-operatively, then the position of the tibial PCL attachment point relative to the reference frame of the navigation system would be known.

A model based approach can be used in which a generic model of a typical knee which includes the attachment point of the PCL to the tibia is morphed or otherwise made patient specific by capturing the position of anatomical features of the patient's tibia using the trackable pointer 156. For example a statistical shape model including the tibial PCL attachment position and captured anatomical data for the patient's tibia may be used to instantiate a patient specific model of the patient's tibia.

Hence, the femur includes a trackable marker and the attachment point of the PCL to the femur has been determined and therefore the position of the attachment point of the PCL to the femur can be determined by tracking the position of the femur. Similarly, the tibia includes a trackable marker and the attachment point of the PCL to the tibia has been determined and therefore the position of the attachment point of the PCL to the tibia can be determined by tracking the position of the femur. Hence, by tracking the position of the tibia and the femur, changes in the length of the PCL can be determined.

At 212, the patient's leg is placed in extension, as illustrated in Figure 1. Then, at least a posterior drawer test is applied to the patient's knee at 214 in which the tibia is moved posteriorly with respect to the femur. In some embodiments, an anterior-posterior drawer test may be used, but only the PCL length data from the posterior part of that test would be used. More specifically, at 214, the surgeon pushes the lower leg away from them in the posterior direction in the sagittal plane and the tracking system captures the position of the femur and the position of the tibia and stores the maximal posterior displacement of the tibia relative to the femur. Then, at 216, the knee angle is changed to a next value, for example by increasing the knee angle by 10°. The method is repeated, as illustrated by process flow line 218, and the posterior drawer test is then repeated at 214 for the new knee angle. Hence, the method repeats to obtain at least posterior displacement data for the tibia relative to the femur as a function of knee angle from full extension through to a flexion angle of at least 90° and in some cases up to about 120°. In some embodiments a lesser range of angles and fewer angle values may be used. For example, the range of angles may be from 30° to 90° and posterior drawer test data may be captured at the angles of and 90° which may be sufficient.

Then at 220 a range of motion test is carried out on the knee in an unstressed state in order to determine the extent to which the femur moves relative to the anterior-posterior axis of the tibia over the range of motion of the knee joint. As will be appreciated, the native condyles of the femur do not have a constant radius and the articulating surfaces of the tibia are not flat and have a complex geometry. Hence, the approximate axis of rotation about the medial-lateral axis of the knee joint can move along the anterior-posterior axis as the knee moves between flexion and extension. Hence, at 220, the knee is articulated over its range of motion between extension and flexion and the positions of the femur and tibia are tracked using the trackable markers 150, 152 and captured by the CAS system. The CAS system then processes the femur position data and the tibia position data as a function of angle to determine how the femur moves relative to the tibia in the anterior-posterior direction of the tibia as a function of knee angle. This provides the native rollback of the patient's knee. Generally, "rollback" may be considered how much the femur moves posteriorly relative to the tibia as the knee bends from extension into flexion.

At 222, the posterior drawer test data is used to determine the maximum PCL length available over a range of knee angles. As the PCL is relatively stiff it is likely to reach close to its maximum length during the posterior drawer test. Hence, the maximum PCL length is an indication of the length of PCL available to the knee and against which the PCL length arising from planned implant positions may be compared. This may provide an indication whether the required PCL length resulting from the current plan is likely to be too much greater (and hence potentially resulting in a lax PCL) or too much less (and hence potentially resulting in a tight PCL) than the native maximum PCL length over the range of knee angles.

An initial plan for the femoral implant and tibial implant has already been established and includes various positions and angles relative to the native femur and tibia respectively. For the planned femoral implant position and the planned tibial implant position, and using virtual models of the femoral implant and the tibial implant at those positions, the inferior-superior distances between the femur and the tibia can be calculated over a range of knee angles. A more accurate determination of the PCL length would also incorporate relative movement of the femur and tibia in the anterior-posterior direction. In one embodiment the relative positions of the tibia and femur from the passive range of motion test at 220 may be used so that the resulting positions of the femur and the tibia as a function of knee flexion angle can be determined by the CAS system. Alternatively, a fixed amount of rollback over the knee angle may be used, e.g. 1mm, 2mm, 3mm or 4mm in total over the knee flexion angle range of, for example, 30° to 90°, or greater as illustrated in the Figures. As the attachment position of the PCL to the femur and to the tibia are known, the distance between those positions as a function of flexion knee angle, which is effectively the length of the PCL as a function of knee angle, can also be determined by the CAS system. Hence, at 224, for a current knee plan, the required length of the PCL for one or more amounts of rollback can be determined as a function of knee flexion angle. One or more required PCL lengths and the maximum PCL length available, as determined at 222, may be output to the user.

From the output of the comparison of the required PCL length to the maximum PCL length available as a function of knee flexion angle, for the currently planned femoral and tibial implant positions, the surgeon may determine at 226 whether any modification to the femoral and/or tibial plan may be appropriate for example to reduce the laxity and/or to reduce the likely tension in the PCL at some knee flexion angle or range of knee flexion angles.

Various factors and approaches may be considered and/or used by the surgeon as appreciated by a person of ordinary skill in the art and s discussed below by way of non-limited examples. The contact point between the femur and tibia may be used as a datum to measure rollback. As the femur flexes, the change in relative position of the femoral and tibial attachment points means that the required PCL length could increase. For a certain amount of femoral flexion, for example approximately 90°, the required PCL length to join the femoral and tibial attachment points may decrease as the femur moves posteriorly relative to the tibia. If the amount of tibial slope (in the anterior to posterior direction) is increased, then the required length of the PCL will decrease more quickly as the femur moves posteriorly relative to the tibia. Removing more posterior bone from the femur (which effectively shifts the femoral implant component anteriorly) will reduce the required PCL length with the femur in flexion, for example at approximately 90°. Removing more distal bone from the femur (which effectively shifts the femoral implant component proximally) will reduce the required PCL length with the femur in extension.

At 226, the surgeon may determine that some change in the plan is desired, for example changing the anterior-posterior slope of the tibial component and may input some new planning data or information into the CAS system. The method then returns to 224, at which for the modified implant plan, the required PCL length for one or more roll back amounts as a function of knee flexion angle is again determined and output to the surgeon.

In an alternative embodiment, generally the same methodology may be applied, but instead the amount of rollback required in order to ensure that the maximum PCL length available is not exceeded may be output.

The modification of the plan may be repeated as indicated by process flow line 228 until the surgeon is satisfied with the implant plan, at this stage of the procedure, and then the remainder of the knee replacement procedure may continue at 230 in a generally conventional manner and using the currently modified plan. It will be appreciated that the plan may be further modified subsequently during the remainder of the procedure 230 for other reasons, if appropriate. However, the method allows an intra-operative check of the PCL length arising from a plan to be carried out without any cuts being made to the native bone stock of the tibia or femur and/or the placement of any trial components. It may be far more difficult to modify a previously made cut in order to take into account the PCL after trialling than it is to take into account the PCL when planning an initial cut, e.g. adding some a-p slope to the initial tibial cut in order to reduce the resulting PCL length at greater flexion angles. Hence, predicting the likely required PCL length for planned positions and comparing that to the maximum PCL length available, before trialling may help to avoid or reduce the need for reworking or cuts and/or releasing of ligaments to try and correct PCL laxity or tension, if at all possible.

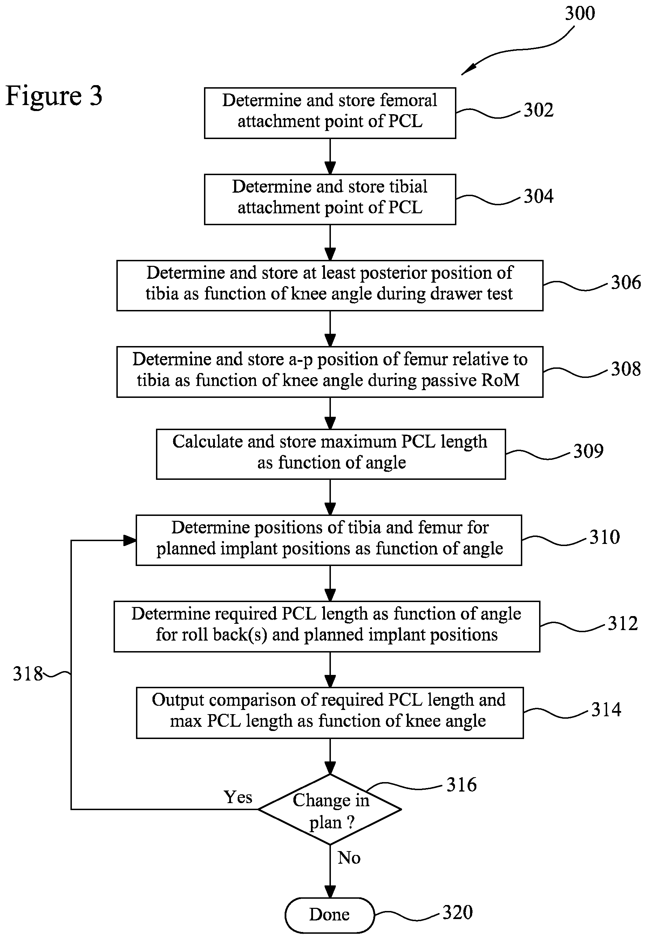

With reference to Figure 3 there is shown a process flow chart illustrating a computer implemented data processing method 300 carried out by the CAS system 104 in parallel with the method illustrated in Figure 2. The data processing method 300 may be implemented as suitable software operating on a general purpose computer, or as a combination of software and hardware. As discussed above the tracking system 102 may track the positions of the patient's femur and tibia and the trackable pointer and provide position data to the CAS system 104 which stores the position data for processing. Hence, the data processing operations carried out by method 300 may use the position data obtained from the tracking system 102.

At 302, the position data from the tracking system is used to determine and store the position of the attachment point of the PCL to the patient's femur in parallel with registration of that point at 208 in Figure 2. Similarly, at 304, the position data from the tracking system is used to determine and store the position of the attachment point of the PCL to the patient's tibia in parallel with registration of that point at 210 in Figure 2. Hence, the CAS system 104 can now determine the position at which the PCL attaches to the femur (which is a measured or registered point) and the position at which the PCL attaches to the tibia (which is an estimated or calculated point) within the reference frame of the tracking system.

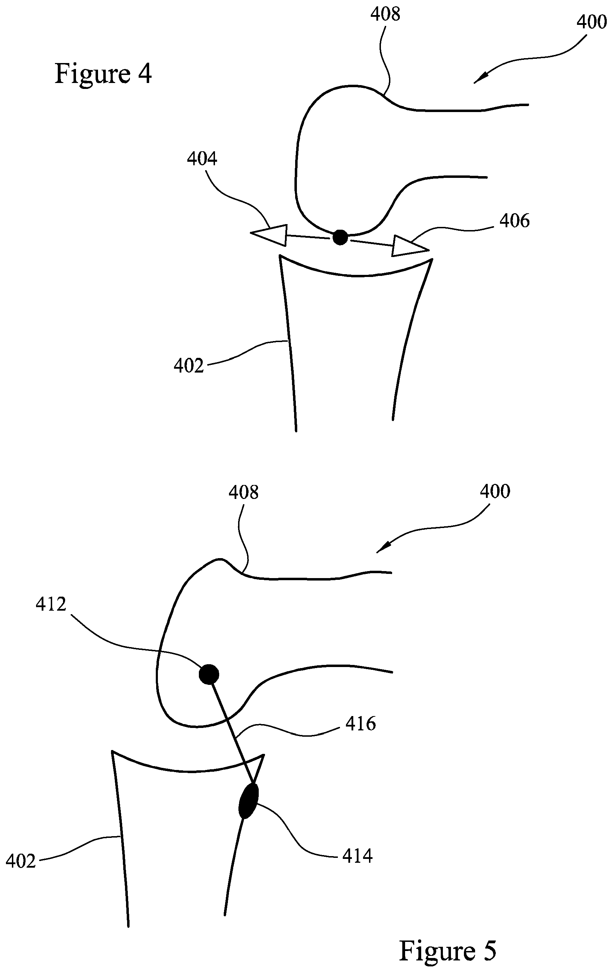

At 306, in parallel with 212, 214 and 216, tracking data for the femoral tracking array 150 and the tibial tracking array 152 is processed to determine the current knee flexion angle, based on the angle between the femoral axis and the tibial axis, and also the position of the tibia relative to the femur along the anterior-posterior axis during the drawer test. As illustrated in Figure 4, which shows a view of the sagittal plane of the patient's knee 400 at a particular knee flexion angle, close to 90°, during application of an anterior and posterior drawer test, the patient's tibia 402 moves in an anterior 404 and a posterior direction 406 relative to the patient's femur 408. The tracking data is processed to determine the maximum anterior position and the maximum posterior position of the tibia for a current knee flexion angle. When the knee flexion angle is changed, the maximum anterior position and the maximum posterior position of the tibia is again determined from the tracking data. Alternatively, only a posterior drawer test may be applied as illustrated by arrow 406.

Figure 5 illustrates the measured attachment point 412 on the femur and the calculated or otherwise derived attachment point 414 on the tibia of the PCL 416. Similarly to Figure 4, Figure 5 shows a view of the sagittal plane of the patient's knee 400 at a particular knee flexion angle, close to 90°, during application of a posterior drawer test, and in which the patient's tibia 402 has moved to a maximal distance in the posterior direction relative to the patient's femur 408 under the force applied by the surgeon and causing the PCL 416 to extend by a certain amount. Hence, at 306, the tracking data is processed to determine the maximum posterior position of the tibia 402 for a current knee flexion angle. When the knee flexion angle is changed, the tracking data for the femoral tracking array 150 and the tibial tracking array 152 are processed to determine the new knee flexion angle and the maximum posterior position of the tibia is again determined from the tracking data.

At 308, in parallel with 220, tracking data for the femoral tracking array 150 and the tibial tracking array 152 is processed to determine the current knee flexion angle, based on the angle between the femoral axis and the tibial axis, and also the relative positions of the tibia and femur along the anterior-posterior axis of the tibia during the passive range of motion test 220. As discussed above, the knee joint is not a simple rotation about an axis with a fixed position, but rather the position of the axis about which the knee rotates effective moves along the anterior-posterior axis of the tibia with the knee flexion angle. That is, the femur tends to move posteriorly relative to the tibia along the anterior-posterior axis of the tibia (generally referred to as roll back). Hence, the tracking data captured during 220 is processed to determine how the femur moves relative to the tibia along the anterior-posterior axis as the knee flexion angle is varied to determine the native roll back of the patient's knee.

At 309 the maximum PCL length of the patient's native knee is estimated over a range of knee flexion angles using the posterior drawer test data from 306 and the tibial and femoral attachment points of the PCL from 302 and 304 to calculate and store the maximum or available PCL length as a function of knee angle for the patient's native knee. The maximum PCL length may be determined at any time after the posterior drawer test data is available and hence in some embodiments may be determined immediately after 306.

In practice, the native PCL wraps a little around the bone and so it is the distance 416 between the attachment points 412, 414 which is used as a surrogate for the actual length of the native PCL. Hence, herein an estimate of, or approximation to, the length of the PCL is used based on the straight line distance between the femoral and tibial attachment points, rather than the exact length of the PCL which, as noted above, is not a straight line. However, the determined estimates of the maximum PCL length and required PCL length are sufficient to provide useful feedback to the surgeon. In the following maximum PCL length and required PCL length will be used generally as short hand, but it will be understood that these are actually estimates of the exact lengths.

At 310, a current planned position for the femoral implant and a current planned position for the tibial implant stored by the CAS system together with virtual models of the femoral implant and the tibial implant are used to calculate the positions of the femur and the tibia as a function of knee flexion angle. As noted above, the position of the axis of rotation can move along the anterior-posterior axis during flexion of the knee and hence the amount of roll back, or position of the femur relative to the tibia along the anterior-posterior axis, as a function of knee flexion angle as determined at 308 is also used at 310 to reflect how the femur and tibia move relative to each other during knee flexion. Hence, at the end of 310 an accurate determination of the relative positions of the femur and tibia for a current planned femoral implant and tibial implant position as a function of knee flexion angle has been determined. This may be repeated for various different amounts of roll back, including the native roll back determined at 308 and/or for fixed amounts of total roll back, e.g. 1mm, 2mm, 3mm or 4mm.

Then at 312, a prediction of the required length of the PCL as a function of knee flexion angle is calculated using the result of 310 and the attachment position of the PCL to the femur, as determined at 302, and the attachment position of the PCL to the tibia, as determined at 304, and stored. Then at 314, a comparison of the predicted required PCL length, determined at 312 for one or more roll back amounts, and the maximum PCL length, determined at 309, as a function of knee flexion angle may be output to the user, for example in a graphical form.

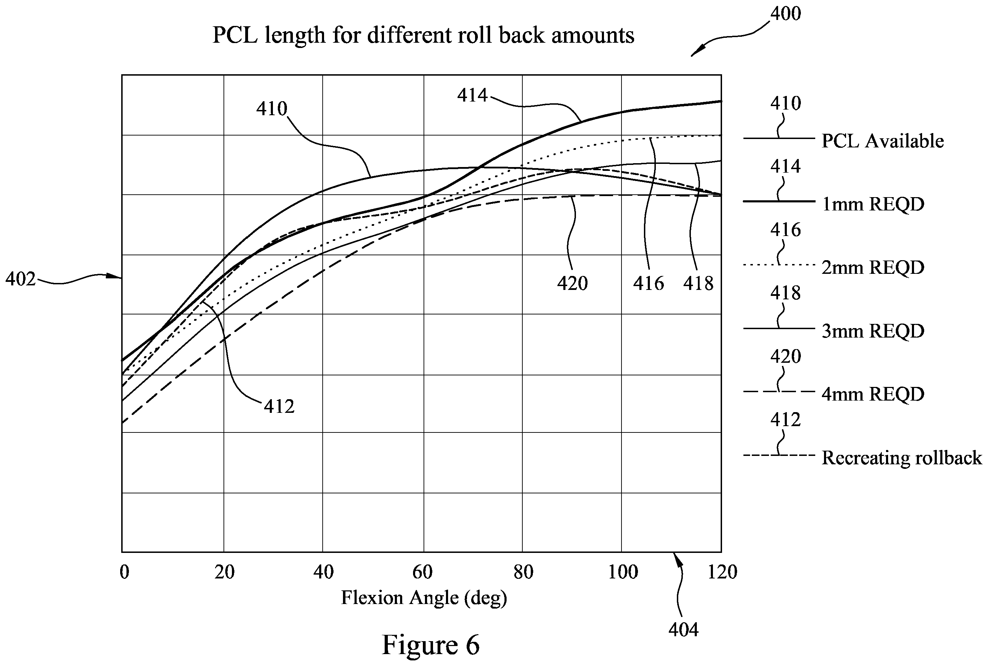

Additionally, or alternatively, at 312, the amount of rollback which will ensure that the required PCL length does not exceed the maximum PCL length available may be determined. Then at 314 that determined amount of roll back may be output, for example in a graphical form or simply as a numerical value or range of values, either additionally to or instead of the output described above and below. For example, as illustrated in Figure 6 (discussed below) it may be determined that an amount of rollback of at least 4mm will ensure that the required PCL length does not exceed the maximum PCL length available over the range of flexion angles for the current planned implant positions and this value may be output to the user.

Figure 6 shows a graphical output 400 in the form of a plot of PCL length in mm on axis 402 as a function of knee flexion angle on axis 404 over the range of approximately 0° to 120°. As illustrated in Figure 6, line 410 shows the variation in the available or maximum PCL length 410 as a function of knee flexion angle. Line 412 shows the PCL length required for the current plan and to recreate or reproduce the native roll back as dashed line 412. Lines 414, 416, 414 and 418 show the PCL lengths required for the current plan and for 1mm, 2mm, 3mm and 4mm of roll back respectively. As can be seen, the available PCL length is sufficient for all the different roll back amounts up to about 70°. At higher knee flexion angles, the required PCL length for the currently planned implant positions and roll backs of 1mm, 2mm and 3mm are greater than the maximum PCL length available 410 and so will likely give rise to significant PCL tension at flexion angles greater than 70 °. However, the 4mm roll back and native roll back result in required PCL lengths above 70° either less than or similar to the maximum PCL length up to about 120°. However, the 4mm roll back required PCL length is less than PCL max for most knee angles less than about 100° and so may give rise to some laxity for lower knee flexion angles.

As discussed above, in some embodiments an indication of the amount of rollback ensuring that the required PCL length is less than PCL max may also be output, and which in this example may be at least 4mm.

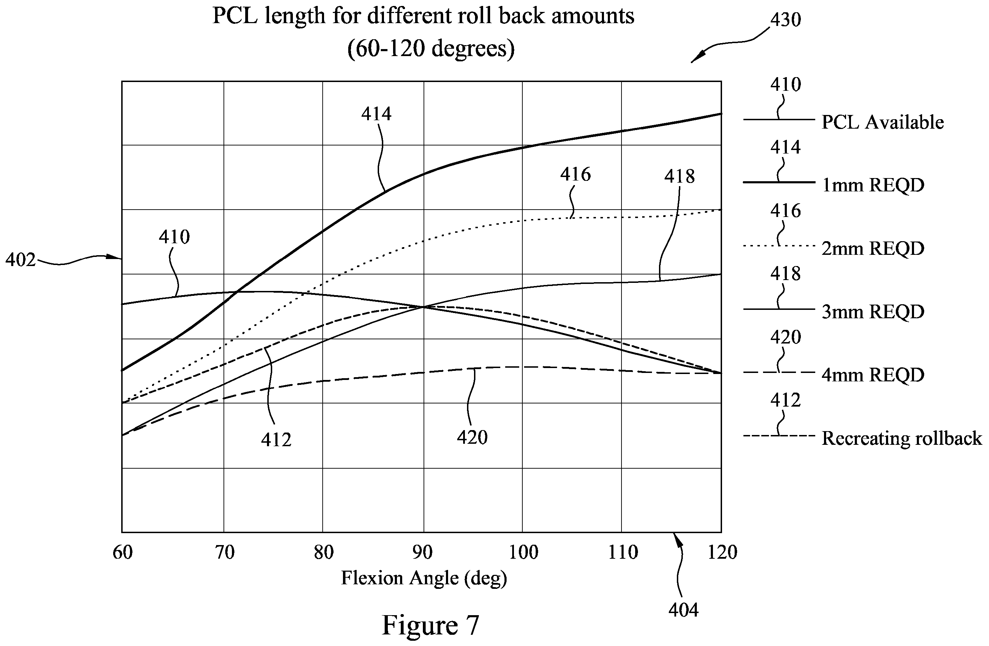

Figure 7 which shows an output 430 similar to Figure 6 but limited to the knee flexion angle range of 60° to 120°, being the range over which the PCL is more involved in knee joint stability. Indeed, knee flexion angles from 60° and greater tend to be more important as these are the knee angles at which the PCL starts to engage and be more involved.

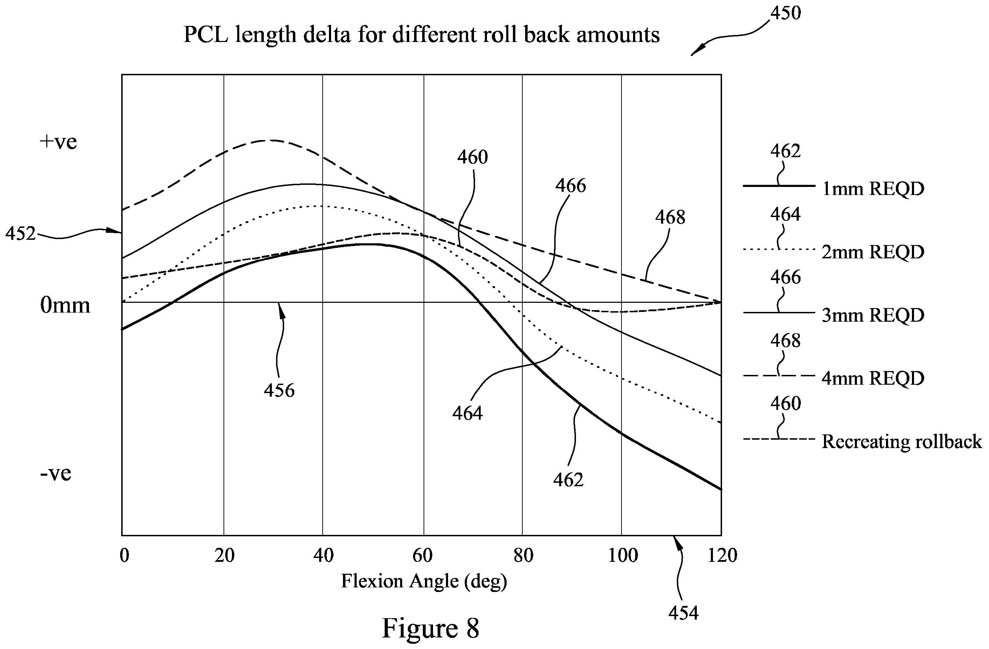

Figure 8 shows a further output 450 which may additionally or alternatively be displayed at 314. Output 450 is a graph of the difference between PCL maximum and PCL required (i.e. PCL max. - PCL req.) on axis 452 as a function of knee flexion angle on axis 454. Line 456 corresponds to zero difference and values above line 456 are positive values of axis 452 and values below are negative values of axis 452. Dashed line 460 shows the difference between PCL maximum and the required PCL length for recreating the native roll back. Similarly lines 462, 464, 466 and 468 show the difference between PCL maximum and the required PCL length for recreating roll backs of 1mm, 2mm, 3mm and 4mm respectively. As will be appreciated, those portions of the lines having positive values correspond to a required PCL length less than the maximum PCL length and hence potential PCL laxity whereas those portions of the lines having negative values correspond to a required PCL length greater than the maximum PCL length and hence potential PCL tightness. Values sufficiently close to line 456 may be considered an acceptable surgical outcome whereas those too far from line 456 may be considered unacceptable and hence modification of the plan or further surgical mitigations may be considered, such as tissue release. It will largely be a matter of surgeon choice, guided by the outputs of the method, as to what amount of rollback to relieve PCL tension may be too great or not.

Hence, at 226 the surgeon may input some new or modified planning data. For example, the PCL tension may be reduced by changing the slope of the tibial tray, for example by modifying the angle of the planned tibial cut and/or by changing the thickness of the tibial component or a tibial insert. Hence, if the user enters some change in the planning data or information into the CAS system at 316, then process flow returns as illustrated by process flow line 318 to 310. Hence, at 310, the positions of the femur and tibia arising from the implants are determined again, but using the modified planning data, and then at 312, the predicted required PCL lengths are determined again using the changed positions of the femur and tibia. As will be appreciated the maximum PCL length will not change. Hence, at 314, the modified predicted PCL lengths for each roll back amount and the maximum PCL length as a function of knee flexion angle may be output again. The modified implant plan may give rise to a required PCL length which is less than the maximum PCL length over the relevant part of the range of knee flexion angles thereby reducing PCL tension and/or closer to the maximum PCL length than previously and hence helping to reduce PCL laxity. Hence, the resulting PCL tension and/or PCL laxity for the modified plan is less likely to exceed the maximum PCL tension as determined form the drawer test and therefore the modified planned implant positions may be considered better.

Various options may be available to the surgeon at or after 316. For example, the maximum PCL length can be increased by releasing the fibres if modifications to the plan are unlikely to address PCL tension. This would likely be done during 320 with a trial in place though. Hence, a required tight PCL from the output 400, 430 would be an indication that releases may be required later in the procedure.

Moving the femoral cut positions at 316 will also change the distance between the femur and the tibia and therefore have an effect on required PCL length.

The other soft tissues of the knee also come into effect, so these would also limit the movement of the knee bones and mean that the PCL data is likely to be more useful at about 60° to 90° degrees where the PCL it is the predominant anterior-posterior restraint.

The surgeon may also balance the PCL tension with other aspects of surgery, such as flexion extension gap balance, medial and lateral balance, etc.

Hence, the planned implant positions may be modified in various ways and any number of times and the process may loop, as illustrated by return line 318, until the surgeon or user determines at 226 that no further modifications to the plan are currently needed. Hence, at 316, when it is determined that no further changes to the planned implant position are currently to be made, the process 300 completes and the current planned implant positions are stored. It will be appreciated that the plan may be modified subsequently if appropriated during the remainder of the surgical procedure 230.

As discussed above, there are various changes available to the surgeon, such as tibial slope, distal femoral cut position, insert thickness, and others, which all have an effect on PCL tension, which in turn has an effect on how much rollback is required in order to prevent the PCL from being too tight. Some optimal, or preferred, value or range of values for rollback may be established, for example empirically or by experiments, and that value or range of values, for example 2-3mm of rollback at 90° of flexion, may then be used by the surgeon as part of their planning of implant position during the method and using the outputs of the method.

In this specification, example embodiments have been presented in terms of a selected set of details. However, a person of ordinary skill in the art would understand that many other example embodiments may be practiced which include a different selected set of these details. It is intended that the following claims cover all possible example embodiments.

Any instructions and/or flowchart steps can be executed in any order, unless a specific order is explicitly stated. Also, those skilled in the art will recognize that while one example set of instructions/method has been discussed, the material in this specification can be combined in a variety of ways to yield other examples as well, and are to be understood within a context provided by this detailed description.

While the disclosure is amenable to various modifications and alternative forms, specifics thereof have been shown by way of example in the drawings and described in detail. It should be understood, however, that other embodiments, beyond the particular embodiments described, are possible as well. All modifications, equivalents, and alternative embodiments falling within the scope of the appended claims are covered as well.

## Claims

1. A non-transitory computer readable medium storing instructions executable by a data processor to carry out a data processing method comprising the following steps:
(i) determining a femoral attachment position of a posterior cruciate ligament to a femur of a knee of a patient;
(ii) determining a tibial attachment position of the posterior cruciate ligament to a tibia of the knee of the patient;
(iii) determining a displacement of the tibia relative to the femur along an anterior-posterior axis of the tibia of the patient for each of a plurality of knee angles, corresponding to a posterior drawer test being applied to the knee of the patient;
(iv) determining the position of the femur and the tibia of the knee of the patient arising from a planned femoral implant position and a planned tibial implant position for each of the plurality of knee angles;
**characterized in that** the method further comprises:
(v) determining a maximum length of the posterior cruciate ligament as the distance between the femoral attachment position and the tibial attachment position for each of the plurality of knee angles using the determined displacement, the femoral attachment position and the tibial attachment position;
(vi) determining a rollback of the femur relative to the tibia along an anterior-posterior axis of the tibia for each of the plurality of knee angles;
(vii) determining a required length of the posterior cruciate ligament for each of the plurality of knee angles based on
(a) the distance between the femoral attachment position and the tibial attachment position for each of the plurality of knee angles using the determined positions of the femur and the tibia arising from the planned femoral implant position and the planned tibial implant position, the femoral attachment position, and the tibial attachment position, and
(b) an amount of rollback required to prevent the required length of the posterior cruciate ligament exceeding the maximum length of the posterior cruciate ligament; and
(viii) outputting a comparison of the required length of the posterior cruciate ligament and the maximum length of the posterior cruciate ligament as a function of knee angle.

2. The non-transitory computer readable medium storing instructions executable by the data processor to carry out the data processing method of claim 1, wherein the roll back corresponds to a fixed amount of roll back of the knee.

3. The non-transitory computer readable medium storing instructions executable by the data processor to carry out the data processing method of claim 1 or claim 2, further comprising:
receiving a change to the planned femoral implant position and/or a change to the planned tibial implant position, and repeating (v), (vi), (vii) and (viii) using the changed planned femoral implant position and/or the changed planned tibial implant position.

4. The non-transitory computer readable medium storing instructions executable by the data processor to carry out the data processing method of claim 1, wherein the amount of rollback is determined for a femoral flexion angle of at least 60°.

5. The non-transitory computer readable medium storing instructions executable by the data processor to carry out the data processing method of any of claims 1 to 4, wherein outputting the comparison of the required length of the posterior cruciate ligament and the maximum length of the posterior cruciate ligament as a function of knee angle comprises outputting a graphical display of the required length of the posterior cruciate ligament and the maximum length of the posterior cruciate ligament as a function of knee angle.

6. The non-transitory computer readable medium storing instructions executable by the data processor to carry out the data processing method of any of claims 1 to 5, wherein the plurality of knee angles are within the range of 60° of flexion to 90° of flexion.

7. The non-transitory computer readable medium storing instructions executable by the data processor to carry out the data processing method of any of claims 1 to 6, wherein the number of the plurality of knee angles is at least three.

8. A data processing apparatus (104) comprising:
a processor; and
the non-transitory computer readable medium of any preceding claim.

9. A computer assisted surgery system (100) including the data processing apparatus (104) of claim 8.

10. The computer assisted surgery system (104) of claim 9, further comprising:
a tracking system (102); and/or
a surgical robot (106).

## Patentansprüche

1. Ein nichtflüchtiges computerlesbares Medium, das Anweisungen speichert, die von einem Datenprozessor durchgeführt werden können, um ein Datenverarbeitungsverfahren auszuführen, das die folgenden Schritte beinhaltet:
(i) Bestimmen einer Oberschenkelbefestigungsposition eines hinteren Kreuzbandes an einem Oberschenkel eines Knies eines Patienten;
(ii) Bestimmen einer Schienbeinbefestigungsposition des hinteren Kreuzbandes an einem Schienbein des Knies des Patienten;
(iii) Bestimmen einer Verschiebung des Schienbeins relativ zu dem Oberschenkel entlang einer Anterior-Posterior-Achse des Schienbeins des Patienten für jeden von einer Vielzahl von Kniewinkeln, entsprechend einem auf das Knie des Patienten angewandten hinteren Schubladentest;
(iv) Bestimmen der Position des Oberschenkels und des Schienbeins des Knies des Patienten, die sich aus einer geplanten Oberschenkelimplantatposition und einer geplanten Schienbeinimplantatposition für jeden der Vielzahl von Kniewinkeln ergibt;
**dadurch gekennzeichnet, dass** das Verfahren ferner Folgendes beinhaltet:
(v) Bestimmen einer maximalen Länge des hinteren Kreuzbandes als den Abstand zwischen der Oberschenkelbefestigungsposition und der Schienbeinbefestigungsposition für jeden der Vielzahl von Kniewinkeln unter Verwendung der bestimmten Verschiebung, der Oberschenkelbefestigungsposition und der Schienbeinbefestigungsposition;
(vi) Bestimmen eines Rollbacks des Oberschenkels relativ zu dem Schienbein entlang einer Anterior-Posterior-Achse des Schienbeins für jeden der Vielzahl von Kniewinkeln;
(vii) Bestimmen einer erforderlichen Länge des hinteren Kreuzbandes für jeden der Vielzahl von Kniewinkeln basierend auf
(a) dem Abstand zwischen der Oberschenkelbefestigungsposition und der Schienbeinbefestigungsposition für jeden der Vielzahl von Kniewinkeln unter Verwendung der bestimmten Positionen des Oberschenkels und des Schienbeins, die sich aus der geplanten Oberschenkelimplantatposition und der geplanten Schienbeinimplantatposition, der Oberschenkelbefestigungsposition und der Schienbeinbefestigungsposition ergeben, und
(b) einem Betrag des Rollbacks, der erforderlich ist, um zu verhindern, dass die erforderliche Länge des hinteren Kreuzbandes die maximale Länge des hinteren Kreuzbandes überschreitet; und
(viii) Ausgeben eines Vergleichs der erforderlichen Länge des hinteren Kreuzbandes und der maximalen Länge des hinteren Kreuzbandes als eine Funktion des Kniewinkels.

2. Nichtflüchtiges computerlesbares Medium, das Anweisungen speichert, die von dem Datenprozessor durchgeführt werden können, um das Datenverarbeitungsverfahren gemäß Anspruch 1 auszuführen, wobei der Rollback einem festen Betrag des Rollbacks des Knies entspricht.

3. Nichtflüchtiges computerlesbares Medium, das Anweisungen speichert, die von dem Datenprozessor durchgeführt werden können, um das Datenverarbeitungsverfahren gemäß Anspruch 1 oder Anspruch 2 auszuführen, ferner beinhaltend:
Empfangen einer Änderung der geplanten Oberschenkelimplantatposition und/oder einer Änderung der geplanten Schienbeinimplantatposition und Wiederholen von (v), (vi), (vii) und (viii) unter Verwendung der geänderten geplanten Oberschenkelimplantatposition und/oder der geänderten geplanten Schienbeinimplantatposition.

4. Nichtflüchtiges computerlesbares Medium, das Anweisungen speichert, die von dem Datenprozessor durchgeführt werden können, um das Datenverarbeitungsverfahren gemäß Anspruch 1 auszuführen, wobei der Betrag des Rollbacks für einen Oberschenkelbeugungswinkel von mindestens 60° bestimmt wird.

5. Nichtflüchtiges computerlesbares Medium, das Anweisungen speichert, die von dem Datenprozessor durchgeführt werden können, um das Datenverarbeitungsverfahren gemäß einem der Ansprüche 1 bis 4 auszuführen, wobei das Ausgeben des Vergleichs der erforderlichen Länge des hinteren Kreuzbandes und der maximalen Länge des hinteren Kreuzbandes als eine Funktion des Kniewinkels das Ausgeben einer grafischen Anzeige der erforderlichen Länge des hinteren Kreuzbandes und der maximalen Länge des hinteren Kreuzbandes als eine Funktion des Kniewinkels beinhaltet.

6. Nichtflüchtiges computerlesbares Medium, das Anweisungen speichert, die von dem Datenprozessor durchgeführt werden können, um das Datenverarbeitungsverfahren gemäß einem der Ansprüche 1 bis 5 auszuführen, wobei die Vielzahl von Kniewinkeln innerhalb des Bereichs von 60° Beugung bis 90° Beugung liegt.

7. Nichtflüchtiges computerlesbares Medium, das Anweisungen speichert, die von dem Datenprozessor durchgeführt werden können, um das Datenverarbeitungsverfahren gemäß einem der Ansprüche 1 bis 6 auszuführen, wobei die Anzahl der Vielzahl von Kniewinkeln mindestens drei beträgt.

8. Eine Datenverarbeitungsvorrichtung (104), die Folgendes beinhaltet:
einen Prozessor; und
das nichtflüchtige computerlesbare Medium gemäß einem der vorhergehenden Ansprüche.

9. Ein computergestütztes Chirurgiesystem (100), das die Datenverarbeitungsvorrichtung (104) gemäß Anspruch 8 umfasst.

10. Computergestütztes Chirurgiesystem (104) gemäß Anspruch 9, das ferner Folgendes beinhaltet:
ein Verfolgungssystem (102); und/oder
einen chirurgischen Roboter (106).

## Revendications

1. Un support non transitoire lisible par ordinateur stockant des instructions exécutables par un processeur de données pour réaliser un procédé de traitement de données comprenant les étapes suivantes :
(i) la détermination d'une position de fixation fémorale d'un ligament croisé postérieur à un fémur d'un genou d'un patient ;
(ii) la détermination d'une position de fixation tibiale du ligament croisé postérieur à un tibia du genou du patient ;
(iii) la détermination d'un déplacement du tibia par rapport au fémur le long d'un axe antéro-postérieur du tibia du patient pour chacun d'une pluralité d'angles de genou, correspondant à l'application d'un test du tiroir postérieur au genou du patient ;
(iv) la détermination de la position du fémur et du tibia du genou du patient résultant d'une position d'implant fémoral planifiée et d'une position d'implant tibial planifiée pour chacun de la pluralité d'angles de genou ;
**caractérisé en ce que** le procédé comprend en outre :
(v) la détermination d'une longueur maximale du ligament croisé postérieur en tant que distance entre la position de fixation fémorale et la position de fixation tibiale pour chacun de la pluralité d'angles de genou en utilisant le déplacement déterminé, la position de fixation fémorale et la position de fixation tibiale ;
(vi) la détermination d'un recul *(rollback)* du fémur par rapport au tibia le long d'un axe antéro-postérieur du tibia pour chacun de la pluralité d'angles de genou ;
(vii) la détermination d'une longueur requise du ligament croisé postérieur pour chacun de la pluralité d'angles de genou sur la base
(a) de la distance entre la position de fixation fémorale et la position de fixation tibiale pour chacun de la pluralité d'angles de genou en utilisant les positions déterminées du fémur et du tibia résultant de la position d'implant fémoral planifiée et de la position d'implant tibial planifiée, la position de fixation fémorale, et la position de fixation tibiale, et
(b) d'une quantité de recul requise pour empêcher que la longueur requise du ligament croisé postérieur n'excède la longueur maximale du ligament croisé postérieur ; et
(viii) l'émission en sortie d'une comparaison de la longueur requise du ligament croisé postérieur et de la longueur maximale du ligament croisé postérieur en fonction de l'angle de genou.

2. Le support non transitoire lisible par ordinateur stockant des instructions exécutables par le processeur de données pour réaliser le procédé de traitement de données de la revendication 1, dans lequel le recul correspond à une quantité fixe de recul du genou.

3. Le support non transitoire lisible par ordinateur stockant des instructions exécutables par le processeur de données pour réaliser le procédé de traitement de données de la revendication 1 ou de la revendication 2, comprenant en outre :
la réception d'un changement de la position d'implant fémoral planifiée et/ou d'un changement de la position d'implant tibial planifiée, et la répétition de (v), (vi), (vii) et (viii) en utilisant la position d'implant fémoral planifiée changée et/ou la position d'implant tibial planifiée changée.

4. Le support non transitoire lisible par ordinateur stockant des instructions exécutables par le processeur de données pour réaliser le procédé de traitement de données de la revendication 1, dans lequel la quantité de recul est déterminée pour un angle de flexion fémorale d'au moins 60°.

5. Le support non transitoire lisible par ordinateur stockant des instructions exécutables par le processeur de données pour réaliser le procédé de traitement de données de n'importe lesquelles des revendications 1 à 4, dans lequel l'émission en sortie de la comparaison de la longueur requise du ligament croisé postérieur et de la longueur maximale du ligament croisé postérieur en fonction d'un angle de genou comprend l'émission en sortie d'un affichage graphique de la longueur requise du ligament croisé postérieur et de la longueur maximale du ligament croisé postérieur en fonction d'un angle de genou.

6. Le support non transitoire lisible par ordinateur stockant des instructions exécutables par le processeur de données pour réaliser le procédé de traitement de données de n'importe lesquelles des revendications 1 à 5, dans lequel la pluralité d'angles de genou se situent dans la plage allant de 60° de flexion à 90° de flexion.

7. Le support non transitoire lisible par ordinateur stockant des instructions exécutables par le processeur de données pour réaliser le procédé de traitement de données de n'importe lesquelles des revendications 1 à 6, dans lequel le nombre de la pluralité d'angles de genou est d'au moins trois.

8. Un appareil de traitement de données (104) comprenant :
un processeur ; et
le support non transitoire lisible par ordinateur de n'importe quelle revendication précédente.

9. Un système de chirurgie assistée par ordinateur (100) incluant l'appareil de traitement de données (104) de la revendication 8.

10. Le système de chirurgie assistée par ordinateur (104) de la revendication 9, comprenant en outre :
un système de suivi (102) ; et/ou
un robot chirurgical (106).
